# EUROPEAN PATENT APPLICATION

(11) **EP 3 588 087 A1**
(43) Date of publication of application: **01.01.2020**
(21) Application number: 17897766.6
(22) Date of filing: 30.08.2017
(51) Int. Cl.: G01N 33/53, G01N 33/543, G01N 37/00

(54) **BIOMATERIAL IMMOBILIZING METHOD AND USES THEREOF**

(30) Priority: 24.02.2017 JP 2017033646
(71) Applicant: Mitsubishi Gas Chemical Company, Inc., Tokyo 100-8324 (JP)
(72) Inventor: TASHIRO, Hideo, Tokyo 113-0024 (JP); NODA, Kohki, Tokyo 113-0024 (JP); TASHIRO, Tomoko, Tokyo 113-0024 (JP)
(74) Representative: Müller-Boré & Partner Patentanwälte PartG mbB
(86) International application number: PCT/JP2017/031281
(87) International publication number: WO 2018/154814

(57) **Abstract**

In one embodiment of the present invention, provided are novel biomaterial immobilizing method having excellent immobilization ability and uses thereof, the method being characterized in that: an immobilization carrier having a surface modification specific to the biomaterial to be immobilized is used; the material to be immobilized is pre-immobilized to the surface of the immobilization carrier; a surface layer for maintaining the immobilization carrier on a biomaterial immobilizing substrate is provided; and the immobilization carrier is stamped in the shape of a spot on the surface layer.

## Description

### Technical Field

The present invention relates to a biological material immobilization method for stably immobilizing a biological material such as a protein, a peptide or a nucleic acid on a substrate. In particular, the present invention can be preferably used in a method comprising immobilizing a biological material on a substrate, then developing an antigen-antibody reaction, and then examining and analyzing this antigen-antibody reaction. In particular, the present invention is preferably used in a biochip for use in examination and diagnosis, which utilizes an antigen-antibody reaction and is used in allergy tests, tumor marker tests, and the like.

### Background Art

In microarray-type measurement methods in which many types of biological materials, such as DNAs and proteins, are disposed, as materials to be immobilized, on the surface of a substrate in the form of small spots, there is a method of spotting and immobilizing materials to be immobilized on a substrate, or a method of synthesizing desired biological materials on a substrate.

In the case of a protein chip for use in an immunoassay involving the microarray-type measurement, an antibody or an antigen needs to be immobilized on a substrate in some form. A DNA chip is mainly applied to synthesis methods, whereas a protein chip and a peptide chip are hardly applied to synthesis methods. That is to say, at present, regarding to the DNA chip, a method of synthesizing nucleotides having a desired sequence on a substrate has been widely adopted. However, under the current circumstances, it is impossible to synthesize a protein on a substrate. In the case of a peptide, it can be synthesized on a substrate, but a peptide having sufficient purity cannot be obtained only by the synthesis thereof. Hence, in the case of such a protein chip and a peptide chip, a method of directly stamping a purified protein or a purified peptide on the surface of a substrate has been mainly applied at the moment.

As such, substrates, which are prepared by introducing functional groups such as carboxy groups (-COOH) or amino groups (-NH₂) into the surface of a glass or a plastic for easy immobilization, are commercially available. Moreover, a photoimmobilization method capable of immobilizing all types of organic matters on a substrate by disposing photofunctional groups thereon has been practicalized.

### Citation List

### Patent Literature

Patent Literature 1: International Publication WO 2005/111618
Patent Literature 2: JP Patent Publication (Kokai) No. 2010-101661 A
Patent Literature 3: International Publication WO 2014/007034

### Non Patent Literature

Non Patent Literature 1: Victor ET Mancilla and Rudolf Volkmer "Peptide arrays on planar supports," Methods in Molecular Biology, 1352, 3-17 (2015)
Non Patent Literature 2: Tinashe B Ruwona, Ryan Mcbride, Rebecca Chappel, Steven R Head, Phillip Ordoukhanian, Dennis R. Burton, and Mansun Law "Optimization of peptide arrays for studying antibodies to hepatitis C virus continuous epitopes" J Immunol Methods. 15, 35-42 (2014)

### Summary of Invention

### Technical Problem

As described above, a method for immobilizing various biological materials to be immobilized on a substrate via a covalent bond has been developed. Moreover, inventions regarding a biological material immobilization agent excellent in terms of a non-specific adsorption-preventing effect, a biological material immobilization method using the aforementioned biological material immobilization agent, and a biological material-immobilizing substrate have also been made. However, a protein chip or a peptide chip having reliability as a diagnostic chip has not yet been established. The greatest reason therefor is difficulty in immobilizing a protein on a substrate with good reproducibility, while maintaining the three-dimensional structure of the protein and physiological function based thereon. In addition, in the case of a peptide that is a much smaller molecule than a protein, it is necessary not only to ensure the amount of the peptide immobilized, but also to align the orientation of molecules, namely, to immobilize the peptide at the N-terminus or C-terminus thereof, in order to keep the functions thereof constant. Moreover, when a protein is finally detected by utilizing the interaction between proteins, such as an antigen-antibody reaction, in a reaction system on a flat substrate, it also becomes an important factor to avoid as much as possible an environment by which two proteins that are reaction partners hit against each other to inhibit the reaction, namely, what is called steric hindrance.

Besides, the non-specific adsorption means that an antibody molecule binds to the surface of organic substances or inorganic substances, such as molecules or polymers other than the partner thereof. Since this is not a chemical reaction, it is referred to as adsorption. Furthermore, the reaction by which an antibody molecule binds to an antigen molecule as a partner thereof is referred to as a specific reaction between an antigen and an antibody.

As a method for immobilizing a protein on a substrate, a photoimmobilization method has been known (Patent Literature 1). This method comprises previously adding a photofunctional group onto a substrate, and then binding the photofunctional group to the amino acid side chain of a protein to be immobilized via a covalent bond, so as to immobilize the protein on a substrate. Specifically, a protein or the like is spotted on a coated surface, which has been coated with a polymer having a photoreactive functional group such as an azide group, and the coated surface is then irradiated with light to cause a radical reaction, so that the material to be immobilized is bound thereto via a covalent bond. This method is characterized in that it does not select a material to be immobilized, but is able to immobilize all types of substances. However, this method has been problematic in that the reaction probability of photofunctional groups is not 100% and immobilization efficiency is not high, in that the density of photofunctional groups is low and thus, is not suitable for immobilization of a low-molecular-weight substance such as a peptide, and also in that the binding site of a molecule to be immobilized cannot be selected.

As a photoimmobilization method for immobilizing a low-molecular-weight substance such as a peptide, there has been proposed a method of utilizing an immobilization agent having at least a photoreactive group and a room temperature-reactive functional group such as an epoxy group in a single molecule thereof (Patent Literature 2). An immobilization agent having a photoreactive group and an epoxy group is mixed into a stamp solution and is then used, so that amino groups contained in a peptide are allowed to react with the immobilization agent on the surface of a substrate at normal temperature, and further, after drying, the reaction mixture is photoimmobilized. However, according to this method, the immobilization reagent is likely to bind to all of the amino groups contained in the peptide, namely, the immobilization reagent is likely to bind to amino groups in a side chain possessed by lysine, one type of amino acid constituting the peptide. Accordingly, it could not be guaranteed that the peptide is immobilized, aligned to the orientation of the surface of the immobilizing substance at the N-terminus of the peptide.

As a method of overcoming the above-described disadvantages, there is the method of Non Patent Literature 2. In this method, the amino group on the N-terminal side is converted to an oxyamino group, so that it is differentiated from the side chain amino group of the peptide, thereby enabling reaction conditions under which only the oxyamino group binds to a functional group on a substrate via an ester bond. However, in order to carry out this method, oxirane-based functional groups need to be previously added onto the surface of the substrate, and further, after completion of the immobilization, unnecessary oxirane-based functional groups need to be inactivated. Moreover, some measures need to be taken to prevent an increase in the background noise caused by non-specific adsorption. Furthermore, upon production of a chip, the reaction between the above-described oxyamino groups and oxirane functional groups is terminated during several minutes until droplets containing the material to be immobilized, which have been stamped on the surface of the substrate, are dried. Hence, such an insufficient reaction time has caused low reaction probability, that is, a small amount of substance immobilized.

In contrast, there is also a method of using microparticles as carriers for immobilization of biomolecules. Specifically, protein molecules and the like are immobilized on surface-modified microparticles according to a desired reaction. The reaction on the microparticle is observed based on color change involving the microparticles, etc. Hence, a single reaction is performed with a single solution, and thus, this method is not suitable for simultaneous determination of multiple types of reactions. In order to overcome this problem, there has also been developed a method which comprises adding fluorescent dyes with different colors and intensities to particles, so as to enable the distinguishing of individual particles, and then performing multiple reaction determination by utilizing flow cytometry.

Patent Literature 3 discloses a method for immobilizing magnetic microparticles on a retention layer, using the magnetic microparticles as microparticles. However, according to the technique described in Patent Literature 3, living body-related molecules used as detection targets are allowed to interact with magnetic microparticles in a solution, and after these living body-related molecules have been captured by the magnetic microparticles, the magnetic microparticles are immobilized on the retention layer and are then fluorescently detected. Specifically, this technique is not different from prior art techniques using microparticles, in that the reaction of capturing the living body-related molecules by the magnetic microparticles is carried out in a solution. Moreover, Patent Literature 3 does not disclose a method for immobilizing functionalized (surface-modified) magnetic microparticles on predetermined positions (spots) with predetermined intervals.

As mentioned above, there are methods comprising immobilizing a material to be immobilized on a microparticle (which is also referred to as a "bead") and then analyzing it in a solution. However, an array technique of immobilizing a material to be immobilized on a substrate such as a slide glass or a chip, mediated by the aforementioned microparticle, has not yet been practicalized (Non Patent Literature 1).

It is an object of the present invention to provide an efficient biological material immobilization method, in which biological materials such as proteins, peptides, nucleic acids, and mixtures thereof can be widely used.

### Solution to Problem

In order to achieve the above-described object, the biological material immobilization method according to one aspect of the present invention is characterized in that it comprises: using an immobilization carrier having surface modification, depending on the type of a biological material, in order to immobilize a material to be immobilized consisting of a biological material, previously immobilizing the material to be immobilized on the surface of the immobilization carrier; then providing a layer for retaining the immobilization carrier on a biological material-immobilizing substrate; and then stamping the immobilization carrier on the layer in the form of spots.

The substrate according to one aspect of the present invention is a substrate on which biological materials are immobilized, comprising:
a plurality of particles on each surface of which a biological material to be immobilized is immobilized; and
an immobilization agent layer, which is provided between each of the plurality of the particles and the surface of the substrate, and is used to immobilize each of the plurality of the particles on the surface of the substrate.

The method for evaluating an interaction with a biological material according to one aspect of the present invention comprises a step of evaluating the interaction of a test substance applied (added) onto the above-described substrate with a biological material immobilized on the substrate.

The method for immobilizing a biological material according to one aspect of the present invention comprises a step of immobilizing a plurality of immobilization carriers, on each surface of which a biological material to be immobilized is immobilized, on the surface of a substrate in the form of spots, via an immobilization agent layer.

The method for producing a substrate according to one aspect of the present invention is a method for producing the above-described substrate, which comprises a step of immobilizing the plurality of the particles on the surface of the substrate via the immobilization agent layer.

### Advantageous Effects of Invention

According to the present invention, there can be provided an efficient biological material immobilization method, in which biological materials such as proteins, peptides, nucleic acids, and mixtures thereof can be widely used.

### Brief Description of Drawings

[Figure 1] Figure 1 is an explanatory view showing a cross-section of the biological material-immobilizing substrate of Example 1 of the present invention.
[Figure 2] Figure 2 is an explanatory view showing a state in which proteins and peptides are immobilized on microparticle carriers in Example 1 of the present invention.
[Figure 3] Figure 3 is an explanatory view showing an example of a process of detecting a biological material as a basis of the present invention. As an example, application to allergy diagnosis, what is called, an antigen-antibody reaction and an analysis result example thereof are shown.
[Figure 4] Figure 4 is an explanatory view showing the enhancement of luminescence signal strength in the case of using bead carriers for immobilizing proteins in Example 1 of the present invention.
[Figure 5] Figure 5 is an explanatory view showing the enhancement of luminescence signal strength in the case of using bead carriers for immobilizing peptides in Example 1 of the present invention.
[Figure 6] Figure 6 is an explanatory view showing a comparison between a one-step stamp method and a stepwise stamp method in Example 2 of the present invention.
[Figure 7] Figure 7 is an explanatory view showing a state in which microparticle carriers are uniformly immobilized on the surface of a substrate, using an ink-jet stamper of Example 2 of the present invention.
[Figure 8] Figure 8 is an explanatory view showing a state in which large-diameter carriers of Example 3 of the present invention are immobilized on the surface of a substrate.
[Figure 9] Figure 9 is an explanatory view showing a state in which magnetic bead carriers of Example 4 of the present invention are immobilized on the surface of a substrate

### Description of Embodiments

### (Summary of Embodiments)

As a result of intensive studies, the present inventors have invented a two-stage immobilization method, by which a non-specific adsorption-preventing agent consisting of a hydrophilic polymer is applied onto a substrate, and thereafter, microparticles (immobilization carriers) on which desired materials to be immobilized, such as a proteins or a peptides, have been immobilized under desired conditions or in desired orientation, are spotted on the coated surface of the substrate on which the above-described non-specific adsorption-preventing agent has been applied, and thereafter, the microparticles themselves are immobilized thereon. The present inventors have found that immobilization can be carried out with good reproducibility and high accuracy according to the two-stage immobilization method, thereby completing the present invention.

Specifically, the present invention provides a two-stage substance immobilization method; which comprises: applying a non-specific adsorption-preventing agent consisting of a hydrophilic polymer on a substrate; then mixing microparticles, on which proteins or peptides have previously been immobilized over a sufficient reaction time, with a photocrosslinking agent having two photoreactive groups in a single molecule thereof; and then spotting the obtained mixture on the surface of the substrate, non-specific adsorption of which has been prevented; or successively spotting the microparticles and the photocrosslinking agent on the surface of the substrate, so that they are laminated on each other.

The conventional immobilization technique has been a one-stage immobilization method, which develops a desired chemical reaction such as an ester bond within a short time until liquid droplets comprising a material to be immobilized spotted on the surface of a substrate (biological material-immobilizing substrate) are dried, so that the material is allowed to bind to the substrate.

In contrast, the present two-stage immobilization method, by which materials to be immobilized have previously been immobilized on microparticles according to a solution reaction, and the microparticles are then immobilized on the substrate using a photocrosslinking agent, enables the reaction under suitable conditions with good reproducibility, so that the present method can stabilize the immobilization rate and can increase the immobilization density.

The diameter of a microparticle is not particularly limited, but it is preferably several hundreds of µm or less, and more preferably 5 µm or less. Thus, the microparticle is significantly higher than a protein, and thus, it ensures reliable immobilization without excessively increasing the density of the photocrosslinking agent. In addition, the microparticle may be an organic microparticle, an inorganic microparticle, a magnetic microparticle, or the like. As an example of such a microparticle, Dynabeads (registered trademark) having a diameter of 2.8 µm, and the like can be used. Depending on the type of a material to be immobilized, such as a protein or a peptide, a microparticle providing an optimal immobilization amount, stability and/or reproducibility may be selected.

Conditions for immobilization of a protein or a peptide on the surface of a microparticle carrier are different from conditions for immobilization of the microparticle carrier on a substrate, and both of the immobilization conditions may be optimized.

The microparticle facilitates reaction operations. Using such microparticles, an antibody may be oriented at the Fc region on a microparticle and may be immobilized thereon, or a peptide may be terminus-specifically immobilized. Thus, using such microparticles, the disadvantages of the conventional immobilization method may be overcome. Accordingly, in the present invention, when a biological material to be immobilized, such as a protein or a peptide, is immobilized on a substrate, the material to be immobilized is not directly immobilized on the surface of the substrate, but a microparticle carrier (bead carrier) is allowed to mediate the immobilization, so that the reaction conditions for immobilization of the material to be immobilized on the carrier, and the reaction conditions of immobilization for the carrier on the substrate, can be each optimized. Thereby, while maintaining the three-dimensional structure of a protein or the physical function based thereon, it becomes possible to immobilize a protein with good reproducibility, or to immobilize a peptide while aligning the orientation of molecules thereof. Therefore, the reliability of a protein chip or a peptide chip used as a diagnostic chip can be improved. Moreover, by utilizing the surface of a microparticle, it can be expected to obtain the effect of significantly increasing the immobilization area, or the effect of reducing the above-described steric hindrance, namely, inhibition of a peptide binding reaction by proteins, as a result that the curved surfaces of microparticles bring on slight angle deviation between protein molecules to be bound to adjacent peptides.

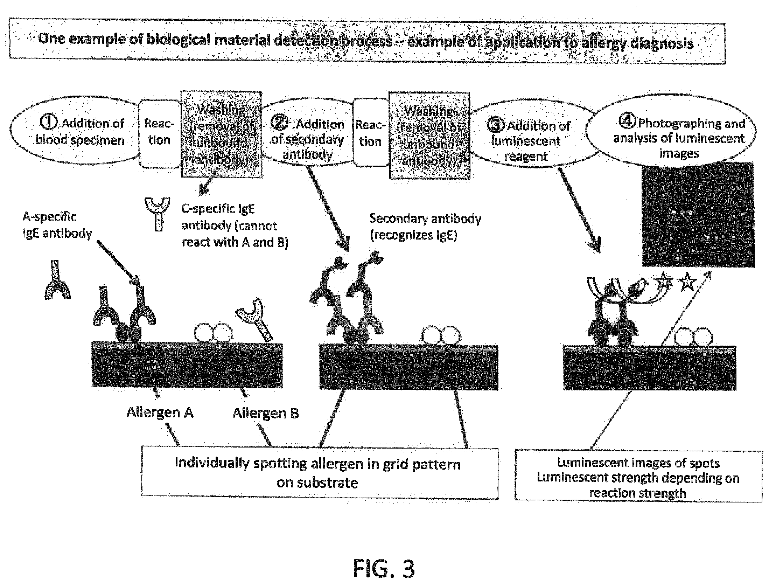

Figure 3 shows an example of a process of detecting an immobilized biological material. As a specific example, a process of detecting an allergen according to an antigen-antibody reaction and analyzing it, using an allergy diagnostic chip, is shown.

In Figure 3, first, a blood specimen (all of whole blood, serum and plasma are available) is injected into a diagnostic chip on which multiple types of allergens have previously been spotted. After the reaction has been carried out for a predetermined period of time, the chip is washed. In this example, it is shown that a specific IgE antibody A reacts with an allergen A and a specific IgE antibody C does not react with allergens A and B. The washing is carried out to remove unreacted specific IgE antibody C. Subsequently, an enzyme-labeled secondary antibody that recognizes the IgE antibody is injected into the chip and is then reacted with the reaction mixture for a predetermined period of time. After completion of the reaction, washing is carried out to remove the secondary antibody, which has not bound to the IgE antibody. Thereafter, a chemiluminescent reagent is injected into the chip. Using a camera, the luminescent image of the spot is photographed and is analyzed. Since the obtained luminescence signal strength is proportional to the strength of the antigen-antibody reaction, the degree of being sensitized to allergy can be measured. It is to be noted that the process of detecting a biological material is not limited to the method shown in Figure 3, but that an optimal method can be selected as appropriate.

Moreover, a protein or a peptide can be immobilized on the surface of a microparticle by various existing methods. For example, a microparticle prepared by modifying the surface of a commercially available microparticle with a carboxy group, an amino group, an epoxy group, etc. (wherein the microparticle is also referred to as a "bead") may also be used.

With regard to beads used in an automatic peptide synthesizer, after unreacted beads have been washed and removed after termination of the synthesis of peptides, the remaining beads can be directly used as peptide carrier microparticles.

If the surface of a microparticle is epoxidated or N-hydroxysuccinimide (NHS)-esterified, and is then reacted with an oxyaminated peptide, N-terminus-selective immobilization also becomes possible. Moreover, a microparticle, to the surface of which streptavidin or neutravidin binds, is reacted with an N-terminus-biotinylated peptide, so that N-terminus-specific immobilization can be efficiently carried out.

By combining a microparticle having an alkyne-introduced surface with an N-terminus-azidated peptide, or by combining a microparticle having an azide group-introduced surface with N-terminus-alkynized peptide, the peptide can be immobilized on the microparticle via an N-terminus-specific covalent bond according to quick chemistry.

It is essential for the above-described non-specific adsorption-preventing agent to have hydrophilic properties. If the non-specific adsorption-preventing agent has hydrophobicity, redundant proteins are adsorbed on the hydrophobic groups, and the accuracy of inspection and analysis is thereby impaired.

According to the present invention, a biological material to be immobilized can be more stably immobilized via a covalent bond. When a protein is immobilized on a substrate according to the method of the present invention, the surface area for immobilization is significantly increased, steric hindrance is prevented, and also, the protein can be immobilized while maintaining the function thereof. Accordingly, it becomes possible to carry out an analysis with high sensitivity, in which the function of the protein is utilized, such as an antigen-antibody reaction or an enzyme reaction. Moreover, a peptide can be reliably immobilized only at the N-terminus. Furthermore, non-specific adsorption is effectively prevented, so that inspection and analysis can be carried out with high accuracy. Therefore, a biological material-immobilizing substrate with high sensitivity and a high signal/noise (S/N) ratio can be obtained. Herein, the term "immobilization" means that a strong chemical bond such as a covalent bond is formed between a microparticle and the surface of a substrate (preferably, a non-specific adsorption-preventing layer), by the mediation of a crosslinking agent (preferably, a photocrosslinking agent), etc. Thus, the term "immobilization" used herein is distinguished from a weak chemical bond such as a hydrogen bond and physical adsorption such as Van der Waals force. Furthermore, the present immobilization is also different from a method of embedding a material to be immobilized into a substrate or into a coating layer consisting of a non-specific adsorption-preventing layer and a photocrosslinking agent in a mechanical dynamic manner.

### (One aspect of the present invention)

Accordingly, the biological material immobilization method according to one aspect of the present invention is characterized in that it comprises: using an immobilization carrier having surface modification, depending on the type of a biological material, in order to immobilize a material to be immobilized consisting of a biological material, previously immobilizing the material to be immobilized on the surface of the immobilization carrier; then providing a layer for retaining the immobilization carrier on a biological material-immobilizing substrate; and then stamping the immobilization carrier on the layer in the form of spots. Besides, "stamping" may also be said to be "spotting."

One aspect of the above-described biological material immobilization method is characterized in that different biological materials are previously immobilized on a plurality of the immobilization carriers, and the plurality of the immobilization carriers are stamped on different positions on the layer. According to such an aspect, the above-described immobilization carriers, to which individual determination functions have been imparted, for example, immobilization carriers to which individual allergens have been bound, are stamped on the layer in the form of spots, so that the results of many types of allergy tests can be simultaneously provided to a patient.

One aspect of the above-described biological material immobilization method is characterized in that organic microparticles, inorganic microparticles, or magnetic microparticles are used as the immobilization carriers. According to such an aspect, by using organic microparticles, inorganic microparticles, or magnetic microparticles as the above-described immobilization carriers, immobilization of biological materials on the immobilization carriers and immobilization of the immobilization carriers on the layer can be carried out, separately. Thus, since the reaction conditions of the two types of immobilization can be each optimized, a high immobilization amount and high stability can be guaranteed, and immobilization can be performed with good reproducibility.

One aspect of the above-described biological material immobilization method is characterized in that a photocrosslinking agent having at least two photoreactive groups in a single molecule is used as a method for immobilizing the immobilization carriers on the biological material-immobilizing substrate. According to such an aspect, by using the photocrosslinking agent having at least two photoreactive groups in a single molecule as a crosslinking agent, a covalent bond can be formed by a simple light irradiation operation, and the immobilization carriers can be stably immobilized on the layer.

One aspect of the above-described biological material immobilization method is characterized in that, as a method of stamping crosslinking agents for crosslinking the layer and the immobilization carriers and the immobilization carriers, in the form of spots on the layer for retaining the immobilization carriers on the biological material-immobilizing substrate, there is adopted a one-step stamp method of stamping a mixed solution prepared by mixing the crosslinking agents with the immobilization carriers, or a stepwise stamp method of first stamping the crosslinking agents and then stamping the immobilization carriers. According to such an aspect, either the one-step stamp method or the stepwise stamp method can be selected. The one-step stamp method comprises simple steps and does not depend on the precision of a spotter. On the other hand, in the case of the stepwise stamp method, the uniformity of spot shapes can be obtained by using a high-precision spotter, and the quality of the image can be enhanced.

One aspect of the above-described biological material immobilization method is characterized in that a water-soluble polymer is applied as a layer for retaining the immobilization carriers on the biological material-immobilizing substrate. According to such an aspect, since a hydrophilic non-specific adsorption-preventing agent is used, the capturing of redundant proteins can be prevented, and the accuracy of inspection and analysis can be improved, in comparison to the case of using a hydrophobic non-specific adsorption-preventing agent.

Next, specific examples (hereinafter referred to as "Examples") of the embodiments of the present invention will be described with reference to drawings. However, these examples are not intended to limit the scope of the present invention.

### Example 1

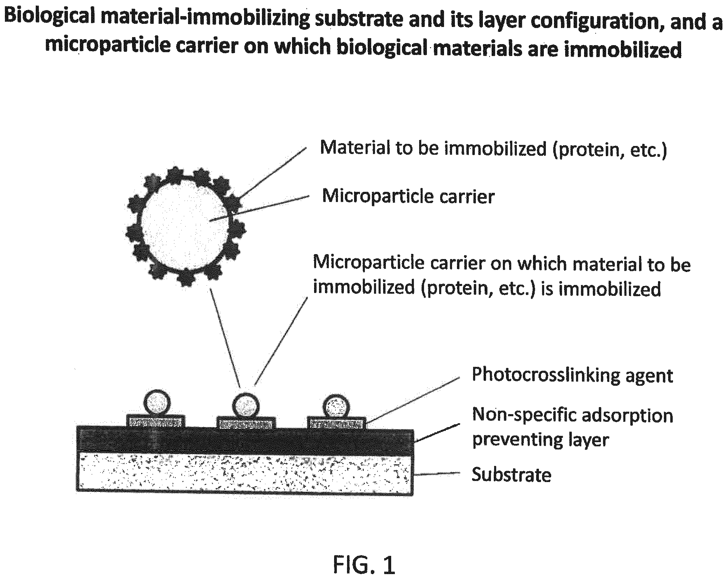

Hereafter, an example of a photoimmobilization method for immobilizing biological material-immobilized microparticles on a substrate, using a photocrosslinking agent (immobilization agent), will be described. Figure 1 shows a configuration of a substrate and a layer produced on the surface of the substrate.

The material of the substrate is not particularly limited. Examples of the material of the substrate may include polystyrene that is widely used in microplates and the like, and light-transmitting resins such as acryl, polycarbonate, polyethylene terephthalate, polypropylene, a cycloolefin polymer (COP), and a cycloolefin copolymer (COC). In addition, a glass and the like can also be used.

As an example of a water-soluble polymer that can be used as a non-specific adsorption-preventing agent, polyethylene glycol (PEG) methacrylate can be used. A water-soluble polymer for preventing non-specific adsorption is known, and it can be produced according to a known production method. Also, some water-soluble polymers are commercially available. Moreover, a method for forming such a non-specific adsorption-preventing layer is also known (for example, Patent Literature 1).

As a crosslinking agent, a photocrosslinking agent having at least two photoreactive groups in a single molecule thereof is preferably used. A covalent bond can be formed by simple light irradiation operations, and microparticles (immobilization carriers) can be stably immobilized on a layer for retaining the immobilization carriers on a biological material-immobilizing substrate. A preferred example of photoreactive groups possessed by the photocrosslinking agent may be an azide group (-N₃), and further, diazidostilbene may be a molecule having two azide groups. Among the photocrosslinking agents used in the present invention, a water-soluble photocrosslinking agent is particularly preferable. The water-solubility herein means that an aqueous solution in a concentration of 0.5 mM or more, and preferably 2 mM or more, can be provided. The concentration of the photocrosslinking agent upon use is not particularly limited, but it is preferably 0.01 mg/mL to 1 g/mL, and more preferably 0.2 mg/mL to 0.2 g/mL.

The biological material immobilized on a substrate according to the present invention is not particularly limited. Examples of the biological material may include a protein, a peptide, a nucleic acid, a lipid, and a sugar. The term "peptide" means a compound in which two or more amino acids bind to one another via a peptide bond. In one example, the biological material may be a polypeptide having 50 or less constitutional amino acid residues. In addition, in one example, the "peptide" may also be said to be a "polypeptide" or a "protein." The "peptide" may further comprise, for example, a structure such as a sugar chain or an isoprenoid group, in addition to a structure formed by the binding of amino acids via a peptide bond.

With regard to azide groups or diazirine groups used as photoreactive groups of the present invention, nitrogen molecules are dissociated from the azide groups or diazirine groups by irradiation with light, and at the same time, nitrogen radicals or carbon radicals are generated. Since these nitrogen radicals or carbon radicals are able to bind, not only to functional groups such as amino groups or carboxy groups, but also to carbon atoms constituting an organic compound, these photoreactive groups are able to be crosslinked to almost all organic matters. Accordingly, the photoreactive groups are able to bind carbon atoms constituting the surface of a microparticle to carbon atoms that are present on a polymer constituting a non-specific adsorption-preventing layer or on the surface of a substrate such as a plastic.

Procedures for producing the layer shown in Figure 1 will be described below.
1) First, a substrate is prepared. The above-described material can be used as a material for the substrate. The thickness of the substrate is not particularly limited, but the substrate preferably has non-deformability to achieve easy handling ability in the production process. The thickness of the substrate is preferably 0.3 mm to 3.0 mm, more preferably 0.5 mm to 1.5 mm, and particularly preferably 0.7 mm to 1.0 mm. In the present example, a flat substrate made of polycarbonate with a thickness of 0.8 mm was used.
2) In order to immobilize a biological material on the surface of the substrate, a coating layer consisting of a non-specific adsorption-preventing layer and a photocrosslinking agent (immobilization agent layer) is spin-coated. The concentration of a water-soluble polymer in a coating solution is not particularly limited, but it may be, for example, 0.0001 to 10 parts by mass, and preferably 0.001 to 1 part by mass. On the other hand, the concentration of the photocrosslinking agent may be, for example, 1 to 20 parts by mass, and preferably 2 to 10 parts by mass, based on the above-described polymer.
3) As a spin coater for coating the above-described coating layer on the substrate, MS-A100 manufactured by MIKASA was used. The film thickness is not particularly limited, but it is generally 0.01 µm to 10 µm, and preferably 0.05 µm to 1 µm. As an example for obtaining a desired film thickness, the rotation speed and the rotation time of the spin coater were set at 800 rpm and 5 sec at the initiation of coating, and thereafter, a film was formed at 5000 rpm for 10 sec. Subsequently, for stabilization of the film formation, the rotation speed and rotation time were set at 1500 rpm and 3 sec at the end of coating, and thereafter, the rotation was terminated. As another method for producing a coating layer, a spray coater can be utilized.
4) After the coating layer has been coated on the substrate, in order to stabilize the coating layer, the coating layer is aged under conditions of a constant temperature and a constant humidity. The temperature is preferably 5°C to 40°C, and more preferably 20°C to 30°C. The humidity is preferably 40% RH to 80% RH, and more preferably 60% RH to 70% RH. The aging period is preferably 1 day to 2 weeks, and more preferably 3 days to 1 week.
5) Microparticles are immobilized on the coating layer according to the below-mentioned method.

In the present example, microparticles, on which desired substances have been immobilized, can be immobilized on a substrate as follows. First, a non-specific adsorption-preventing agent (water-soluble polymer) is coated on a substrate. In the one-step stamp method, a mixture of microparticles and photoimmobilization agents (photocrosslinking agents) is spotted on a non-specific adsorption-preventing agent, is then dried, and is then irradiated with light. In the stepwise stamp method, photoimmobilization agents are first spotted on a non-specific adsorption-preventing agent, then, microparticles to be immobilized are spotted thereon, followed by light irradiation. As a result, a large number of photocrosslinking agents are present around the microparticles, and many covalent bonding bridges are generated between the non-specific adsorption-preventing agent polymers and the microparticles, so that the microparticles can be stably immobilized on the substrate.

An important advantage of the method of using microparticles is that the solution can be easily exchanged with another solution without dilution of the concentration of biomolecules. In order not to damage the functions of biomolecules immobilized on the surfaces of microparticles, the reaction has been generally carried out in a state in which the microparticles are suspended in a solution. As such, there is no need to immobilize microparticles on a substrate, and therefore, a suitable microparticle adhesion method for immobilizing microparticles on a substrate has not been present so far.

For example, an immobilization method of allergen-added microparticles will be described. The present inventors have discovered that photoimmobilizing molecules, which have originally been used for protein molecules can also be used for microparticles. In addition, the inventors have confirmed that, in the case of microparticles having a larger mass than protein molecules, even if the concentration of photoimmobilizing molecules is decreased to approximately 1/1000 in the case of protein molecules, immobilization can be stably carried out.

The reason why allergens have previously been added to microparticles will be explained. In order to align molecules to be immobilized, namely, in order to carry out the reaction specifically at the terminus, it is necessary to adjust the reaction conditions and to carry out the reaction slowly over a considerable period of time. Otherwise, non-specific binding reactions may also occur. Thus, it becomes difficult to sufficiently carry out the reaction until the stamping solution is dried (in general, 5 minutes or shorter). In the present invention, as described in the Examples, the reaction of immobilizing molecules to be immobilized on microparticles was carried out mildly under physiological pH conditions over 6 hours. With regard to immobilization of the microparticles on a substrate, the stamping solution was dried immediately after the stamping (within 10 minutes), and photoimmobilization irradiation was then carried out.

The reason for spotting microparticles will be explained below. As mentioned above, it is most important for peptides to carry out a reaction capable of reliably immobilizing them in constant orientation (for example, at the N-terminus). In addition, it is greatly advantageous in that molecules to be immobilized are immobilized on the entire surface of microparticles, so as to increase the effective surface area and also to increase the immobilized amount.

In the present invention, microparticles could be stably spotted on the substrate by finding out conditions for preventing the condensation or precipitation of microparticles during the step of stamping them on the substrate, such as pH or concentration, as well as surface modification on beads and the size of beads. Specifically, beads with an average diameter of 0.2 µm having epoxy groups on the surface thereof (epoxy beads) were reacted with peptides A or B to be modified, in 25 mM MES (pH 6.0) at 40°C a whole day and a night, while stirring, so that the peptides were immobilized on the beads via amino groups. Moreover, such epoxy beads with an average diameter of 0.2 µm were reacted with a streptavidin protein solution in a 25 mM HEPES solution (pH 7.9) at 37°C a whole day and a night, so as to produce avidin-coated beads. This avidin-coated bead suspension was reacted with a solution of peptides A or B having biotinylated N-terminus at 4°C for 1.5 hours, while stirring, so that the peptides A or B were N-terminus-specifically immobilized on the surface of the beads.

The irradiation light used in the above-described photoimmobilization is light under which photoreactive groups can generate a radical reaction. When azide groups or diazirine groups are used as photoreactive groups, ultraviolet ray with 300 to 400 nm is preferable. The dose of light to be irradiated is not particularly limited, but it is generally approximately 1 mW to 100 mW per cm².

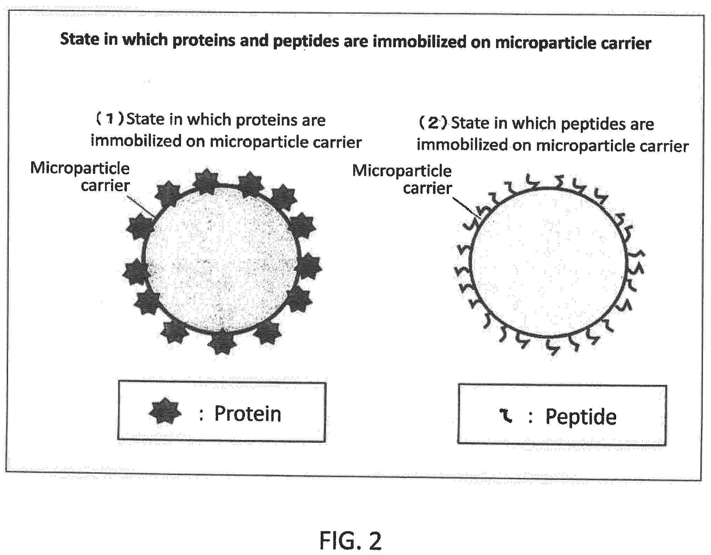

In order to prevent agglutination and/or precipitation occurring in the process of producing a chip, the microparticle carrier for immobilizing a protein or a peptide has a diameter of preferably 2.8 µm or less, and more preferably 1 µm or less. In the present example, microparticles having a diameter 2.8 µm and 0.2 µm were used. Figure 2(1) shows a state in which proteins are immobilized on a microparticle carrier, whereas Figure 2(2) shows a state in which peptides are immobilized on a microparticle carrier.

A difference from Patent Literature 2 will be described. For immobilization of proteins on a microparticle carrier, in general, a method of producing a covalent bond via a primary amino group is used. In the case of a protein having a certain higher-order structure, the side chain amino group or N-terminal amino group of lysine that is only amino acid having an amino group on the side chain, which is present on the surface of a molecule, randomly reacts and binds to a microparticle. In contrast, in the case of a peptide having an amino acid sequence shorter than that of a protein, it is necessary to allow the peptide to bind to a microparticle carrier at the N-terminus, so as to align the orientation. That is, the reaction is controlled such that it is mediated by only the N-terminal amino group contained in all peptides, and thereby, the peptides are immobilized on a microparticle carrier in constant orientation. By doing so, the functions of the peptide, such as, for example, constant epitopic properties of an allergen, can be guaranteed for the first time. In the case of using a reaction that cannot guarantee the terminus binding ability, for example, the binding reaction of an epoxy group (Patent Literature 2) with an amino group, a peptide to be immobilized can bind to a microparticle carrier even mediated by lysine present in the sequence. Depending on the position of such lysine in the sequence, the flexibility of the three-dimensional structure of the peptide is significantly decreased, the original specific binding with proteins such as antibody molecules or receptors becomes impossible, and thereby, the functions of the peptide cannot be guaranteed.

A difference from Patent Literature 1 will be described. Regarding the amount of a photoimmobilization agent, in principle, photoimmobilization agent molecules in an amount equal to or greater than a single molecule to be immobilized are necessary. Therefore, in the case of small molecules such as peptides, the necessary concentration of photoimmobilization agent molecules is extremely high (at a level of mg/mL), and the surface of a substrate is changed to a hydrophobic surface, thereby inducing non-specific adsorption. Thus, this conventional method is not practical. In addition, this method has also been problematic in that the binding sites of molecules to be immobilized cannot be selected. Moreover, as described in the above section regarding the epoxy reaction, since immobilization cannot be carried out in constant orientation, the functions of the peptide cannot be guaranteed.

### Example 2

As described above, microparticles on which proteins or peptides have previously been immobilized are stamped (spotted) on a substrate, which have been coated with polyethylene glycol (PEG) methacrylate.

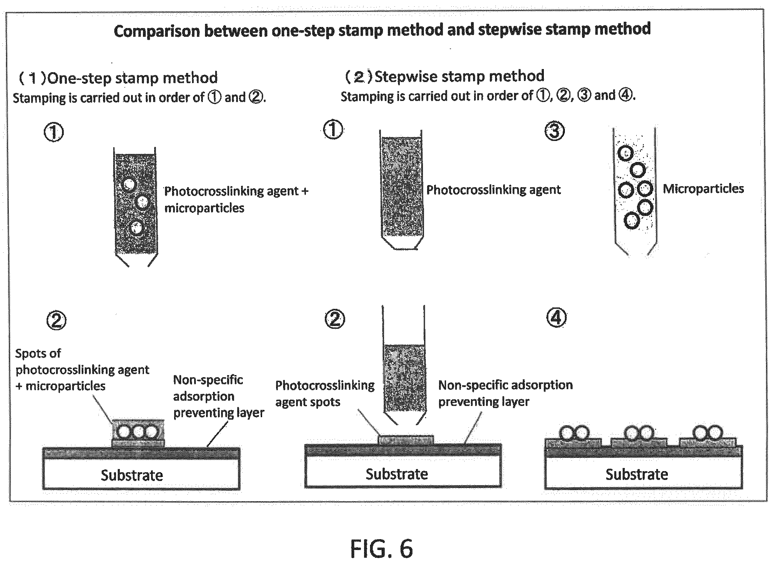

There are two types of stamp methods, namely, a one-step stamp method and a stepwise stamp method. Figure 6 shows a comparison made between the two stamp methods. In the one-step stamp method shown in Figure 6(1), a mixed solution of photocrosslinking agents and beads is stamped on a substrate. In the stepwise stamp method shown in Figure 6(2), photocrosslinking agents are first stamped, and then, a microparticle solution is stamped on the previously stamped photocrosslinking agents. In general, which one of the two stamp methods is used and the results obtained thereby, are different depending on the types of photocrosslinking agents and beads used, and the types of proteins or peptides immobilized on the beads.

In the one-step stamp method, a mixed solution comprising photocrosslinking agents, beads and salts is spotted on a substrate by one step. Thus, the step is simple, and the accuracy of the spotter is not required so much.

In the stepwise stamp method, since photocrosslinking agents that generally do not contain salts are used as a first step, the photocrosslinking agents can be uniformly spotted in an almost round shape on a substrate. Therefore, in the next step, a mixed solution comprising beads and salts can be spotted in a round shape, on or in the spots of the above-described photocrosslinking agents. That is to say, the stepwise stamp method is a technique that aims for the uniformity of the spot shapes. However, this method requires the center accuracy of the spotter. That is, it is the point that the spotting is carried out in the same center in the first step and in the second step. According to this stepwise stamp method, the quality of the image can be enhanced.

Hereafter, an example of immobilization of protein-immobilized beads on a substrate according to the one-step stamp method will be given.
1) First, a substrate coated with a non-specific adsorption-preventing agent was prepared. A ring for storing a solution was provided in the center of the substrate. The ring was made of silicone, and as a dispenser for discharging the silicone, SHOTmini 100 manufactured by Musashi Engineering Inc. was used. The ring was adjusted to have a predetermined inner diameter of 14.4 mm. The outer diameter was determined based on the drawing line width that was determined by the inner diameter of a nozzle of the dispenser. As a result, the outer diameter was 18.8 mm.
2) Proteins were immobilized on a microparticle with a diameter of 0.2 µm as follows.
3) To a suspension of protein-immobilized microparticles with a diameter of 0.2 µm (concentration: 5 mg/mL, 10 mM HEPES, pH 7.0), 1% 4,4'-diazidostilbene-2,2'-disulfonic acid (hereinafter abbreviated as "bisazide") was added as a photocrosslinking agent. The concentration of the photocrosslinking agent was set at 0.01% to 0.1% of the weight of the microparticles.
4) Stamping of Microparticles (Beads)
   In the present example, Genex Arrayer manufactured by Geneqs was used. Ink-Jet Stamper manufactured by Scienion and BioDot (registered trademark) manufactured by BioDot, or products equivalent thereto may also be used. The spot method may be a lattice point-type multipoint dispensing spot method.
5) Drying
   After completion of the spotting, the resultant was dried in a vacuum dryer at room temperature for 10 minutes. When the degree of vacuum reached 0.09 MPa, the valve was closed, and the pump was terminated.
6) Photoimmobilization
   After completion of the drying, the stamped substrate was removed from the dryer, and it was then irradiated with black light for 7 minutes.

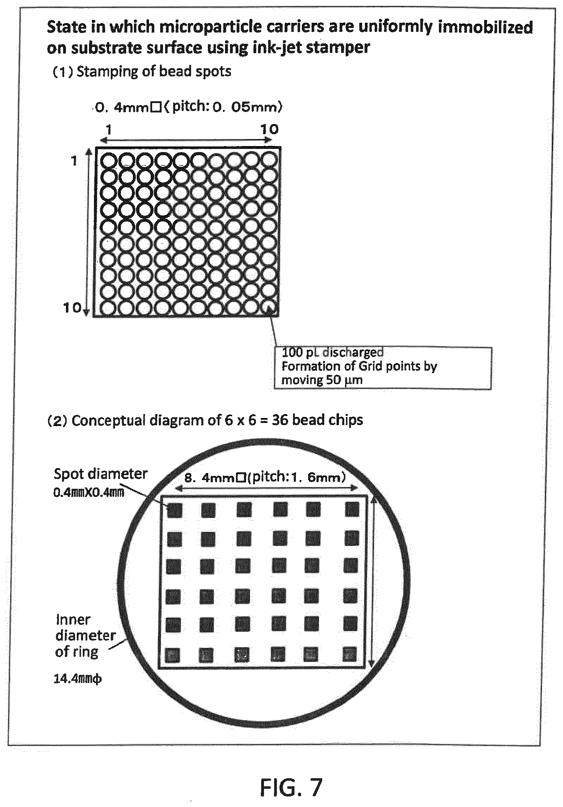

As an example, using an ink-jet stamper, microparticles on which biological materials have been immobilized can be uniformly distributed and immobilized on the surface of a substrate according to the following method. Figure 7 shows the state thereof.

A nozzle with an inner diameter of 50 µm is used, and the discharged amount is set at 100 pL. While the nozzle is moved, for example, to the X-axis direction, the microparticles were discharged with intervals of 50 µm. Thereafter, the nozzle is further moved to the Y-axis direction, and the operation is repeatedly carried out, so that spots with a 0.4 mm square or spots with any other shape can be formed, as shown in Figure 7(1).

Thirty-six types of microparticles, on which different types of substances (allergens) have been immobilized, are prepared, and spots are then formed in positions different from one another, so that diagnostic chips with respect to the 36 types of allergens can be produced, as shown in Figure 7(2). Specifically, spots each having different determination functions can be formed.

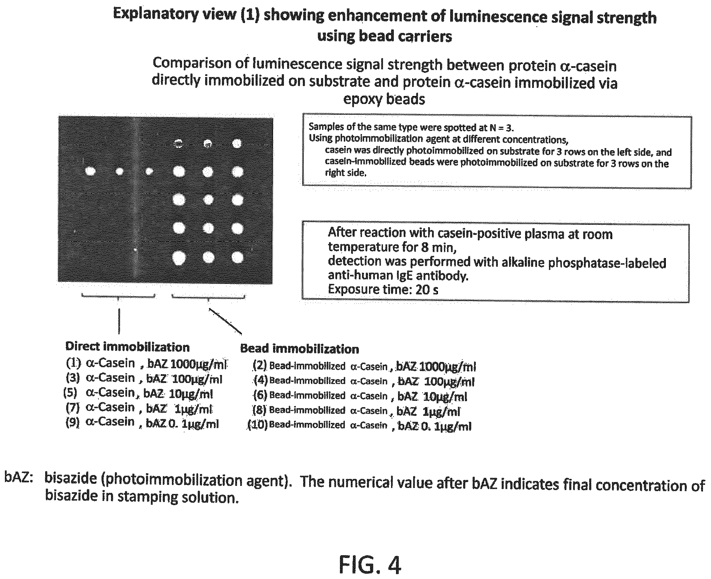

As a more specific example, a spot image based on chemiluminescence shown in Figure 4 will be described.

First, a semiautomatic measuring and/or photographing apparatus was specially manufactured by Precision Shibasaki Co., Ltd., in accordance with the specification provided by the present inventors. Into a lens barrel having vertical driving properties and excellent light shielding properties, a CCD camera with F1.2 manufactured by Nikon (manual focus lens focal length: 50 mm) was placed. Upon photographing, the lens-barrel was closed, and external light was blocked, so that the light of only the spot image was placed into the camera system. Thus, a spot image having a weak strength could be obtained. As image processing software, ChipSolver and SpotSolver, which were specially manufactured by Dynacom Co., Ltd. in accordance with the specification provided by the present inventors, were used.

Hereinafter, an example of immobilization of protein-immobilized beads on a substrate according to the one-step stamp method, namely, procedures for obtaining the image of Figure 4 will be described. In the present example, the case of directly immobilizing proteins on a substrate was compared with the case of immobilizing proteins on beads and then immobilizing the beads on a substrate, in terms of detection strength, namely, luminescence signal strength. In order to make such comparison, a total of 10 types of samples (5 types for each group) were prepared. Hereafter, procedures for preparing a stamping solution will be described.
1) The water-soluble polymer layer (non-specific adsorption-preventing layer) shown in Figure 1 was coated on the surface of a substrate with a size of 25 mm x 25 mm square, and a photoimmobilization agent (coating layer) was then coated thereon, using a spin coater, so as to prepare a chip cassette.
2) A 96-well titer plate was prepared.
3) A 1 mg/mL protein α-casein solution was added into each well (reaction chamber) of the titer plate, so that a total of 10 wells were prepared.
4) 20 µl of protein (1 mg/mL) was injected into wells with odd numbers (1, 3, 5, 7, and 9).
5) 20 µl of a suspension of beads with a diameter of 0.2 µm suspended in 10 mM HEPES (pH7.0) was injected into wells with even numbers (2, 4, 6, 8, and 10).
6) With regard to the concentration of the photoimmobilization agent bisazide, in general, the concentration that is 1/10 of the protein concentration is used as a standard concentration of bisazide (i.e., it is 100 µg/mL with respect to the above-described protein concentration). In the present example, this standard concentration was shifted to a larger value and a smaller value, so that an optimal value was obtained. That is to say, the photoimmobilization agent was injected into Well Nos. 1 and 2 to a final concentration of 1000 µg/mL, into Well Nos. 3 and 4 to a final concentration of 100 µg/mL, into Well Nos. 5 and 6 to a final concentration of 10 µg/mL, into Well Nos. 7 and 8 to a final concentration of 1 µg/mL, and into Well Nos. 9 and 10 to a final concentration of 0.1 µg/mL. Moreover, for the purpose of uniformly equalizing the shape of spots to a round shape, 0.05% Tween (surfactant) and 0.1% polyvinyl alcohol (PVA) were added to the stamping solution.
7) The titer plate, in which the stamping solution was stored, was equipped into Genex Arrayer manufactured by Kaken Geneqs, Inc.
8) First, in the case of direct immobilization, a pin of the stamper was immersed in the stamping solution with Well No. 1, and the stamping solutions stored in wells with odd numbers 1, 3, 5, 7 and 9 were stamped on predetermined positions on the surface of the substrate. The same solution was stamped three times in total with predetermined intervals of 0.3 mm. This is because the reliability of data was increased. Likewise, the stamping stored in Well Numbers 3, 5, 7 and 9 were each stamped three times in total.
9) Next, in the case of bead immobilization, the stamping solutions stored in wells with even numbers 2, 4, 6, 8 and 10 were successively stamped in the same manner as the above 8).
10) Thus, a chip cassette, in which desired stamping solutions had all been stamped, was created.
11) In the drying step, after completion of the spotting, the resultant was dried in a vacuum dryer at room temperature for 10 minutes. When the degree of vacuum reached 0.09 MPa, the valve was closed, and the pump was terminated.
12) In the photoimmobilization step, after completion of the drying, the stamped substrate was removed from the dryer, and it was then irradiated with black light for 7 minutes.
   Thus, a chip cassette, in which 10 types of allergens were mounted in 30 spots in total, was completed.
13) The chip cassette was equipped into the above-described measuring and/or photographing apparatus, and 130 µL of plasma specimen was then injected into the chip cassette by pipetting, followed by measurement and photographing.
14) As a result, the spot image shown in Figure 4 was obtained.

The main allergen of milk, α-casein, and α-casein immobilized on epoxy beads were spotted on a substrate each three times (N = 3) in each immobilization agent concentration, and the plasma of a milk allergy patient was then reacted therewith. Thereafter, an alkaline phosphatase-labeled anti-human IgE antibody, and then, a luminescent reagent as an alkaline phosphatase substrate were reacted therewith. The obtained results are shown in Figure 4.

The luminescence signal strength obtained from the image of Figure 4 is shown in the following Table 1.

**[Table 1]**

| | Comparative Example 1 | | Example 2-1 | |
|---|---|---|---|---|
| Immobilization conditions | Directly immobilized α casein | | Bead-immobilized α casein | |
| Experimental examples (reaction conditions) | Immobilizing agent concentration 0.1 mg/mL | Immobilizing agent concentration 0.1 µg/mL | Immobilizing agent concentration 0.1 mg/mL | Immobilizing agent concentration 0.1 µg/mL |
| Experimental Example 1 (room temp., 8 min) | 60,198 | 25,505 | 79,911 | 115,222 |
| Experimental Example 2 (room temp., 30 min) | 68,828 | 31,661 | 115,622 | 159,108 |

| | | | | |
|---|---|---|---|---|
| Numerical value indicates luminescence signal strength. | | | | |

From the results of Figure 4 and Table 1, it is found that when the concentration of the immobilization agent is low, proteins are not immobilized in the first place. In contrast, if the concentration of the immobilization agent is too high, large quantities of proteins are immobilized, and the reaction with IgE contained in the plasma is inhibited due to steric hindrance. The diameter of a bead carrier is µm order, and thus, since the size of the bead carrier is larger than the size of a protein that is 3 to 10 nm, the amount of the immobilization agent per unit area may be adequately one hundredth or less. It is considered that, in the case of using bead carriers, an expansion in the effective protein-immobilized area and a reduction in the immobilization agent bring on the effect of preventing steric hindrance and the effect of retaining the functions of the protein, thereby resulting in an increase in signal values. Therefore, from the results shown in Figure 4 and Table 1, it is found that low immobilization efficiency, which has been the problem of the photoimmobilization method described in the aforementioned Patent Literature 1 and the like, could be improved.

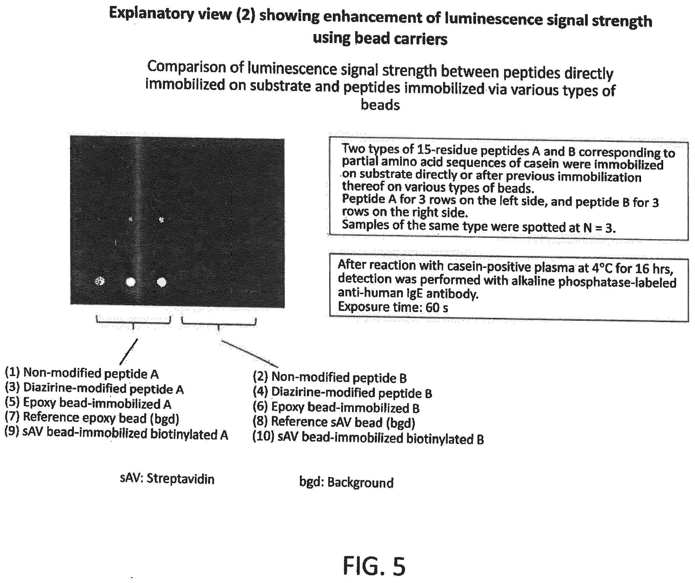

Partial peptides A and B having α-casein different from each other (each consisting of 15 amino acid residues) were directly immobilized on a substrate, or were immobilized on beads and were then immobilized on a substrate. Thereafter, the plasma of a milk allergy patient was reacted with these peptides, and an alkaline phosphatase-labeled anti-human IgE antibody, and then, a luminescent reagent as an alkaline phosphatase substrate were reacted therewith. The obtained results are shown in Figure 5. Herein, the peptide A is a biological material shown with the amino acid sequence LRFFVAPFPEVFGKE, whereas the peptide B is a biological material shown with the amino acid sequence KVPQLEIVPNSAEER.

The luminescence signal strength obtained from the image of Figure 5 is shown in the following Table 2.

**[Table 2]**

| | Comparative Example 2 | | Example 2-2 | | Example 2-3 |
|---|---|---|---|---|---|
| Immobilization conditions | Direct immobilization | | Immobilization on small-diameter beads (diameter: 0.2 µm) | | Immobilization on large-diameter beads (diameter: 2.8 µm) |
| Peptide | Non-modified peptide | Terminal photofuncti onal group-modified peptide (diazirine) | Immobiliz ation on epoxy beads | Immobiliza tion on avidin beads (biotinylate d peptide) | Immobilization on carboxy beads |
| α Casein partial peptide A | 1950.3 ± 205 | 3742.2 ± 410 | 13189.6 ± 1005 | 24982.7 ± 2058 | 11586.7 ± 5460 |
| α Casein partial peptide B | 1893.4 ± 194 | 3594.3 ± 385 | 5332.3 ± 525 | 10125.6 ± 899 | 4952.37 ± 2970 |

| | | | | | |
|---|---|---|---|---|---|
| Numerical value indicates luminescence signal strength. | | | | | |

Besides, with regard to the peptides A and B, the synthesis and purification of non-modified peptides, peptides having an N-terminus modified with the photofunctional group diazirine, and N-terminal biotinylated peptides were carried out by a special synthesis company in accordance with the specification provided by the present inventors.

From the results shown in Figure 5 and Table 2, it was found that the luminescence signal strength obtained in the case of using bead carriers to immobilize the peptides is 3 to 6 times higher than the luminescence signal strength obtained in the case of not using such bead carriers, and thus, the enhancement of the bead carriers could be shown. It is suggested that, in particular, in the case of immobilization of peptides, the use of bead carriers be essential for practicalization. Accordingly, immobilization of small molecules such as peptides, which had been impossible by the photoimmobilization method described in the aforementioned Patent Literature 1, etc., has become possible with the use of bead carriers. At the same time, as mentioned in the subsequent section, the object that is the binding of molecules to be immobilized at specific sites has also been overcome.

With regard to immobilization with beads, from the results shown in Figure 5 and Table 2, it was found that the signal value in the case of immobilizing terminal biotin-modified peptides on avidin beads is 2 times higher than that in the case of immobilizing non-modified peptides on epoxy beads. In the case of using epoxy beads, peptides are immobilized not only at the N-terminus, but also at side chain amino groups of lysine residues contained in the peptides. Hence, it is considered that peptides having different orientations are present on the beads, and thus that the reactivity with an antibody is decreased. In the case of avidin beads, it is understood that peptides are immobilized only at the N-terminus as a result of the specific reaction between avidin and biotin, and thus that the orientation of peptides becomes constant. Therefore, from the results shown in Figure 5 and Table 2, the previous problem that it cannot be guaranteed that peptides are immobilized at the N-terminus thereof on the surface of an immobilizing substance has been overcome.

In the case of Example 2-2 shown in Table 2, small-diameter beads in the stamping solution were hardly precipitated, and the beads were dispersed on the entire spot. As such, it is considered that large luminescence signal strength was obtained from each spot and the standard deviation was also decreased. In the case of Example 2-3, even if the diameter of beads was increased, the signal strength did not change, and further, the standard deviation was increased. This is considered because the concentration of beads in the stamping solution was changed by precipitation of the beads during the stamping step, etc., and thus because the bead concentration was not constant in every spots.

### Example 3

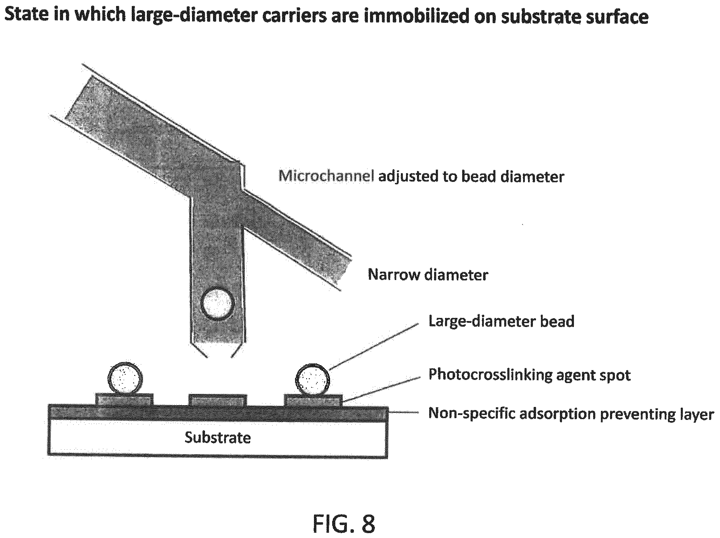

An alternative method of discharging beads, on which proteins or peptides have been immobilized, will be described below. The method is shown in Figure 8. Polymer beads having a nominal diameter of 100 µm are used. First, the beads, together with a solution, are inserted into a capillary having a diameter thicker than the particle diameter. The tip of the capillary has a Y-shaped structure. One end of the Y-shaped structure is a capillary having a diameter slightly thicker than the bead, and a discharging nozzle having pores with the same diameter as that of the bead is provided at the capillary. The diameter of the capillary at the other end of the Y-shaped structure is set to be smaller than the diameter of the bead. Since the capillary with a smaller diameter enables a higher flow rate of the beads than the capillary with a larger diameter, the diameters of the beads can be distinguished from each other, based on it. Hence, only beads with a desired diameter can be immobilized, one by one, on the photocrosslinking agent.

### Example 4

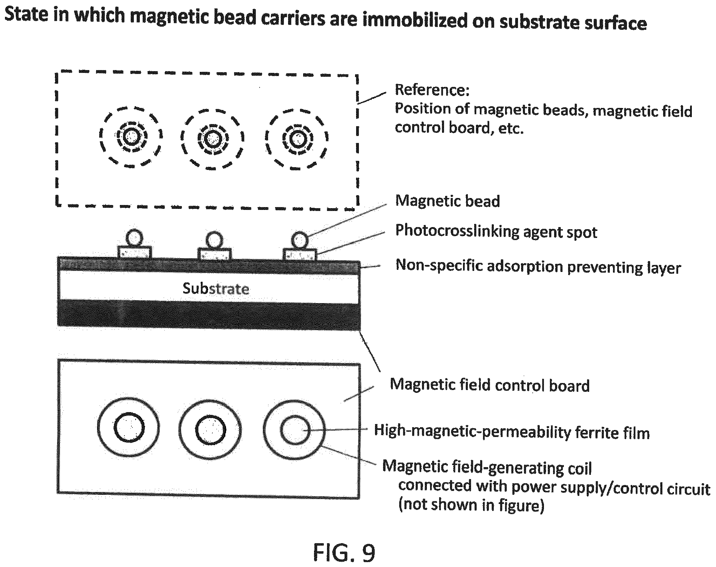

A method of retaining magnetic beads, on which proteins, peptides or the like have been immobilized, on an immobilization substrate, using magnetizing force without agglutination, will be described below. The method is shown in Figure 9. Dynabeads (registered trademark) M-270, having a diameter of 2.8 µm and comprising carboxy groups, is used. A magnetic field-generating mechanism for switching on/off is provided on the back side of the substrate, whereas a retention layer for retaining magnetic beads is provided on the surface of the substrate. First, a dispersed solution of magnetic beads is discharged on the surface of the substrate in a state in which the magnetic field on the back side of the substrate is "off." Since the diameter of a nozzle of a discharging mechanism has been adjusted to the diameter of a magnetic bead according to a microchannel production technique, the magnetic beads are discharged one by one. Subsequently, the magnetic field is turned to "on" to attract the magnetic beads in a solution to the surface of the substrate. The magnetic beads attracted to the substrate are immobilized on the retention layer, and then, the magnetic field is turned to "off." Thereby, the magnetic beads can be retained on the immobilizing substrate without agglutination.

The above-described magnetic field-generating mechanism is required to have a magnetic flux density of approximately 0.1 T (1 KG) that is sufficient for retaining the magnetic beads on the surface of the substrate by magnetizing force. By applying a printed board production technique, a high-permeability thin-film ferrite core and a thin-film coil surrounding the core are formed. By these configurations, a magnetic field-generating mechanism for generating a magnetic flux density, which has a ferrite core in the center of a coil with a diameter of 1 mm or less, is formed. A plurality of the magnetic field-generating mechanisms are disposed, depending on the dimension in which antigens to be spotted are disposed in a grid pattern. A DC power and an on-off control circuit, which are prepared separately, are connected with a plurality of coils,. On/off is controlled depending on the timing of discharging the magnetic beads.

The Examples of the present invention can be preferably used in production of an immunoassay plate on which an antibody or an antigen is immobilized, and also in production of a nucleic acid chip in which DNA or RNA is immobilized on a substrate, a microarray on which a peptide or a protein is immobilized, etc. However, the use of the Examples of the present application is not limited thereto.

Besides, even if a layer for retaining the above-described immobilization carriers on the above-described biological material-immobilizing substrate was not used, it was possible to directly immobilize the immobilization carriers (microparticles) on the substrate by increasing the concentration of the photocrosslinking agent with respect to the immobilization carriers (microparticles) to approximately 10 times greater than in the case of using the retention layer. In contrast, in the above-described embodiment, by using such a layer for retaining the immobilization carriers on the biological material-immobilizing substrate, the concentration of the photocrosslinking agent can be decreased to approximately 1/10.

### Example 5

The present invention also provides the following. It is to be noted that the following description may be applied to the above-described inventions, as appropriate. In addition, the above-described description may also be applied to the following explanation regarding the invention.

Provided is "a substrate on which biological materials are immobilized, comprising:
a plurality of particles on each surface of which a biological material to be immobilized is immobilized; and
an immobilization agent layer, which is provided between each of the plurality of the particles and the surface of the substrate, and is used to immobilize each of the plurality of the particles on the surface of the substrate."

Examples of the biological material may include those as described above. The particles (immobilization carriers) are not particularly limited, but examples of the particles may include those as described above regarding "microparticles." The immobilization agent layer is a layer comprising an agent for immobilizing the particles on the surface of the substrate (immobilization agent). In one example, the immobilization agent is a crosslinking agent that crosslinks the particles and the surface of the substrate. The crosslinking agent is preferably a photocrosslinking agent having at least two photoreactive groups in a single molecule thereof. As a more specific explanation of the photocrosslinking agent, for example, the above description can be referred. For the material of the substrate (biological material-immobilizing substrate) and the like, for example, the above description can be referred.

The immobilization agent layer may be disposed as a plurality of spots on the above-described surface of the substrate. On a single spot, a plurality of the above-described particles may be immobilized, or only one particle may also be immobilized. In one example, a large number of beads (more than several ten thousands of beads) having a diameter of 0.2 µm are immobilized on the surface of the substrate, so that uniformity among the spots and reproducibility can be improved.

The plurality of the particles on each surface of which different biological materials are immobilized may be disposed in the spots different from one another. As specific examples, Example 2 and Figure 7(2) can be referred.

The above-described surface of the substrate is preferably flat and smooth.

The surface of the substrate preferably has a suppressed non-specific adsorption on substances interacting with the biological materials. The embodiment of the suppression of non-specific adsorption is as described above.

The particles may be organic microparticles, inorganic microparticles, or magnetic microparticles.

The biological materials may be either proteins or peptides. In addition, the peptides are preferably immobilized on the particles at the C-terminus or N-terminus thereof.

In one example, the biological materials are allergens.

Provided is "a method for evaluating an interaction with a biological material, comprising:
a step of evaluating the interaction of a test substance applied onto the substrate with a biological material immobilized on the substrate."

In one example of the above-described evaluation method, the test substance is blood (all of whole blood, serum and plasma are available), whereas the biological material is an allergen. According to this evaluation method, if a significant interaction between a test substance and a biological material (allergen) were detected, it would mean that the test substance comprises an antibody reacting against the allergen. That is to say, it becomes an indicator showing that a human or an animal, from which the test substance has been collected, would have allergy against the allergen.

Provided is "a method for immobilizing a biological material, comprising a step of immobilizing a plurality of immobilization carriers, on each surface of which a biological material to be immobilized is immobilized, on the surface of a substrate in the form of spots, via an immobilization agent layer." The form of the immobilization carrier is not particularly limited, but it has, for example, a particulate form.

The above-described immobilization carriers may be organic microparticles, inorganic microparticles, or magnetic microparticles.

In one example, the above-described immobilization agent layer comprises a crosslinking agent that crosslinks the immobilization carriers and the surface of the substrate. Moreover, the crosslinking agent is preferably a photocrosslinking agent having at least two photoreactive groups in a single molecule.

In one example, the step of immobilizing the plurality of the immobilization carriers on the surface of the substrate in the form of spots comprises:
1) applying a liquid containing the plurality of the immobilization carriers and the immobilization agent onto the surface of the substrate in the form of spots; or
2) after application of the immobilization agent on the surface of the substrate in the form of spots, applying the plurality of the immobilization carriers on the surface of the substrate.

In one example, in the above 2), the immobilization carriers are selectively applied to the spots of the immobilization agent applied onto the surface of the substrate.

Provided is "a method for producing the above-described substrate, comprising:
a step of immobilizing the plurality of the particles on the surface of the substrate via the immobilization agent layer."

In one example, the step of immobilizing the plurality of the particles on the surface of the substrate comprises:
1) applying a liquid containing the plurality of the particles and the immobilization agent onto the surface of the substrate in the form of spots, or
2) after application of the immobilization agent onto the surface of the substrate in the form of spots, applying the plurality of the particles onto the surface of the substrate.

In one example, in the above 2), the particles are selectively applied to the spots of the immobilization agent applied onto the surface of the substrate.

The present invention is not limited to each of the aforementioned embodiments, but various modifications may be carried out on the present invention within the scope of claims. Thus, embodiments obtained by appropriately combining technical means disclosed in different embodiments with one another are also included in the technical scope of the present invention. Moreover, the technical means disclosed in individual embodiments can be combined to form novel technical characteristics.

### Industrial Applicability

According to the present invention, there can be obtained a stable immobilization substrate, on which substances to be immobilized can be immobilized while arranging them in terms of sequence or orientation, and in which a non-specific adsorption is suppressed. Thereby, for example, a test substrate on which different types of proteins or peptides are immobilized as a plurality of spots is produced to conduct simultaneous measurement of multiple items, so that a highly accurate and highly reliable diagnostic system can be realized.

## Claims

1. A biological material immobilization method, which comprises: using an immobilization carrier having surface modification, depending on the type of a biological material, in order to immobilize a material to be immobilized consisting of a biological material, previously immobilizing the material to be immobilized on the surface of the immobilization carrier; then providing a layer for retaining the immobilization carrier on a biological material-immobilizing substrate; and then stamping the immobilization carrier on the layer in the form of spots.

2. The biological material immobilization method according to claim 1, wherein different biological materials are previously immobilized on a plurality of the immobilization carriers, and the plurality of the immobilization carriers are stamped on different positions on the layer.

3. The biological material immobilization method according to claim 1 or 2, wherein an organic microparticle, an inorganic microparticle, or a magnetic microparticle is used as the immobilization carrier.

4. The biological material immobilization method according to any one of claims 1 to 3, wherein a photocrosslinking agent having at least two photoreactive groups in a single molecule is used as a method for immobilizing the immobilization carriers on the biological material-immobilizing substrate.

5. The biological material immobilization method according to any one of claims 1 to 4, wherein, as a method of stamping a crosslinking agent for crosslinking the layer and the immobilization carrier and the immobilization carrier, in the form of spots on the layer for retaining the immobilization carrier on the biological material-immobilizing substrate, there is adopted a one-step stamp method of stamping a mixed solution prepared by mixing the crosslinking agent with the immobilization carrier, or a stepwise stamp method of first stamping the crosslinking agent and then stamping the immobilization carrier.

6. The biological material immobilization method according to any one of claims 1 to 5, wherein a water-soluble polymer is applied as a layer for retaining the immobilization carriers on the biological material-immobilizing substrate.

7. A substrate on which a biological material is immobilized, comprising:
a plurality of particles on each surface of which a biological material to be immobilized is immobilized; and
an immobilization agent layer, which is provided between each of the plurality of the particles and the surface of the substrate, and is used to immobilize each of the plurality of the particles on the surface of the substrate.

8. The substrate according to claim 7, wherein the immobilization agent layer comprises a crosslinking agent for crosslinking the particles and the surface of the substrate.

9. The substrate according to claim 8, wherein the crosslinking agent is a photocrosslinking agent having at least two photoreactive groups in a single molecule.

10. The substrate according to any one of claims 7 to 9, wherein the immobilization agent layer is disposed as a plurality of spots on the surface of the substrate.

11. The substrate according to claim 10, wherein the plurality of the particles on each of which different biological materials are immobilized are disposed in the spots different from one another.

12. The substrate according to any one of claims 7 to 11, wherein the surface of the substrate is flat and smooth.

13. The substrate according to any one of claims 7 to 12, wherein the surface of the substrate has a suppressed non-specific adsorption of a substance interacting with the biological material.

14. The substrate according to any one of claims 7 to 13, wherein the particles are organic microparticles, inorganic microparticles, or magnetic microparticles.

15. The substrate according to any one of claims 7 to 14, wherein the biological material is a protein or a peptide.

16. The substrate according to claim 15, wherein the peptide is immobilized on the particles at the C-terminus or N-terminus thereof.

17. The substrate according to any one of claims 7 to 16, wherein the biological material is an allergen.

18. A method for evaluating an interaction with a biological material, comprising:
a step of evaluating the interaction of a test substance applied onto the substrate according to any one of claims 7 to 17 with the biological material immobilized on the substrate.

19. The method according to claim 18, wherein the test substance is blood and the biological material is an allergen.

20. A method for immobilizing a biological material, comprising a step of immobilizing a plurality of immobilization carriers, on each surface of which a biological material to be immobilized is immobilized, on the surface of a substrate in the form of spots, via an immobilization agent layer.

21. The method according to claim 20, wherein the immobilization carriers are organic microparticles, inorganic microparticles, or magnetic microparticles.

22. The method according to claim 20 or 21, wherein the immobilization agent layer comprises a crosslinking agent for crosslinking the immobilization carriers and the surface of the substrate.

23. The method according to claim 22, wherein the crosslinking agent is a photocrosslinking agent having at least two photoreactive groups in a single molecule.

24. The method according to any one of claims 20 to 23, wherein the step of immobilizing the plurality of the immobilization carriers on the surface of the substrate in the form of spots comprises:
1) applying a liquid containing the plurality of the immobilization carriers and the immobilization agent onto the surface of the substrate in the form of spots; or
2) after applying the immobilization agent on the surface of the substrate in the form of spots, applying the plurality of the immobilization carriers on the surface of the substrate.

25. The method according to claim 24, wherein, in the above 2), the immobilization carriers are selectively applied to the spots of the immobilization agent applied onto the surface of the substrate.

26. A method for producing the substrate according to any one of claims 7 to 17, comprising:
a step of immobilizing the plurality of the particles on the surface of the substrate via the immobilization agent layer.

27. The method according to claim 26, wherein the step of immobilizing the plurality of the particles on the surface of the substrate comprises:
1) applying a liquid containing the plurality of the particles and the immobilization agent onto the surface of the substrate in the form of spots, or
2) after applying the immobilization agent onto the surface of the substrate in the form of spots, applying the plurality of the particles onto the surface of the substrate.

28. The method according to claim 27, wherein, in the above 2), the particles are selectively applied to the spots of the immobilization agent applied onto the surface of the substrate.
